# FASCICULE DE BREVET EUROPEEN

(11) **EP 0 371 877 B1**
(45) Date de publication et mention de la délivrance du brevet: **31.03.1993**
(21) Numéro de dépôt: 89403304.2
(22) Date de dépôt: 29.11.1989
(51) Int. Cl.: G01N 15/08, G01N 23/10, E21B 49/00

(54) **Dispositif pour la mesure des écoulements fluidiques à travers un corps poreux**
Vorrichtung zur Messung von Flüssigkeitsströmungen in einem porösen Körper
Device for measuring the flow of fluid through a porous body

(30) Priorité: 30.11.1988 FR 8815661
(43) Date de publication de la demande: 06.06.1990
(73) Titulaire: ELF AQUITAINE PRODUCTION, 92400 Courbevoie (FR)
(72) Inventeur: Morineau, Yves, F-64160 Morlaas (FR); Begani, Rino, F-64121 Montardon (FR)
(74) Mandataire: Boillot, Marc

(56) Documents cités:
- GB-A- 2 185 114
- US-A- 4 537 063
- US-A- 4 543 821
- US-A- 4 599 891
- US-A- 4 753 107

## Description

La présente invention concerne un dispositif pour la mesure des écoulements de fluides à travers un corps poreux et, plus particulièrement, à travers des carottes de milieux poreux provenant des champs pétrolifères.

Afin d'apprécier les performances des différents mécanismes de production à l'échelle microscopique et de déterminer les paramètres quantitatifs destinés à alimenter les modèles numériques de prévision de production, on effectue couramment des écoulements d'un fluide ou des déplacements de plusieurs fluides dans les carottes.

Des dispositifs de réalisation d'écoulements de fluide à travers des carottes ou des échantillons sont connus depuis fort longtemps. On peut se référer utilement à l'article de G.L. Hassler, R.R. Rice et E.H. Leeman, intitulé "Investigation on the recovery of oil from standstone by gas-drive" (Trans. Aime. Vol. 118, page 116, 1936).

Les dispositifs de mesure comportant une cellule d'écoulement en plastique, généralement utilisés en "conditions de laboratoire", sont de plus en plus abandonnés au profit des dispositifs comportant une cellule en acier dont l'épaisseur permet une tenue en pression de plusieurs centaines de bars, à des températures pouvant dépasser 150°C. Dans ces derniers dispositifs, ce sont des carottes ou échantillons réels qui sont disposés dans la cellule, et des fluides réels provenant du champ pétrolifère qui sont utilisés. Les mécanismes de production peuvent donc être étudiés dans les conditions réelles du champ.

Toutefois, ces dispositifs, tout au moins ceux que l'on utilise en "conditions de gisement" parce qu'ils font appel à des carottes et à des fluides réels, permettent uniquement d'évaluer les performances des mécanismes étudiés à partir de bilans matières entre ce qu'il y a initialement dans la carotte étudiée, ce qu'on y injecte, et ce qu'on produit.

En effet, il n'est pas possible de déterminer ce qui se produit à l'intérieur de la carotte, entre le point d'injection et le point de production. En particulier, on ignore tout des distributions locales de saturation, de la forme et des déformations des fronts de déplacement qui peuvent être générées par des hétérogénéités ou des rapports de mobilité des fluides défavorables ou toutes autres causes. Cela rend hasardeuses et même parfois impossibles beaucoup d'interprétations.

De manière à suivre les écoulements à l'intérieur des carottes, on utilise, de plus en plus -dans les dispositifs de mesures travaillant en condition de laboratoire avec cellule en plastique- la gammagraphie, c'est-à-dire la mesure de l'atténuation d'un rayonnement X ou gamma par la matière traversée, atténuation qui est fonction de la densité des éléments rencontrés.

Comme les cellules métalliques des dispositifs de mesure en condition de gisement ne sont pas suffisamment transparentes aux rayons X ou gamma, il est proposé de remplacer la cellule métallique par une cellule composite beaucoup moins absorbantes.

Une telle cellule est décrite dans GB-A-2.185.114. Cette cellule, correspondant au préambule de la revendication 1, est destinée à un travail sous scanner. Elle a été conçue sans tirants de maintien des pistons, de manière à être transparente sous un angle de 360 degrés. Elle est réalisée par enroulements de filaments constitués de fibres de carbone enrobées de résine, lequel enroulement se fait selon deux angles différents pour que la cellule qui est donc réalisée de deux couches, une couche intérieure et une couche externe, tienne aux pressions radiales et axiales.

Le dispositif décrit dans le document GB-A-2.185.114 présente des inconvénients même s'il constitue un progrès non négligeable par rapport aux dispositifs de mesure antérieurs. En premier lieu, une couche constituée par un composite à base de fibre de carbone et de résine a une durée de vie et une efficacité qui sont inférieures à celles des cellules métalliques, en raison des efforts auxquels elle est soumise, et ce malgré l'épaisseur importante qu'on peut lui conférer. En second lieu, il faut noter des difficultés liées à l'étanchéité. Un revêtement interne d'étanchéité de la fibre en élastomère est utilisé. On sait que l'efficacité et la durée de vie de ce revêtement sont problématiques, ainsi que la mise en oeuvre. De plus, ce revêtement ne permet pas d'avoir des surfaces métalliques rectifiées aux normes conventionnelles pour accepter des joints toriques classiques. Il faut donc mettre en oeuvre des jonctions étanches complexes entre le corps de cellule et les pistons, ce qui, outre les inconvénients liés à une fiabilité discutable, interdit toute souplesse dans le choix de la longueur de l'échantillon de roche à étudier.

La présente invention a pour but de remédier aux inconvénients précités et a pour objet un dispositif de mesure susceptible d'être utilisé en gammagraphie et en conditions de gisement et conçu pour travailler sous un dispositif plus modeste de gammagraphie, c'est-à-dire un angle de transparence plus faible, 140° par exemple.

A cet effet, le dispositif de mesure selon l'invention comprend un corps tubulaire en deux parties dont la partie externe est perméable aux rayons gamma, un support fixe d'échantillon disposé à une autre extrémité du corps, une gaine souple disposée à l'intérieur du corps tubulaire, l'échantillon étant disposé dans la gaine, des moyens d'injection d'au moins un fluide à une extrémité du corps tubulaire et à travers ledit échantillon, caractérisé en ce que ledit corps tubulaire est revêtu sur sa paroi latérale d'une chemise métallique de faible épaisseur et perméable audit rayonnement formant la partie interne, le corps tubulaire étant maintenu entre des tirants de maintien, le dispositif comprenant de plus un piston introduit en partie à l'autre extrémité dudit corps tubulaire, l'échantillon étant disposé entre le piston et le support.

Un avantage de la présente invention réside dans le fait que la chemise métallique permet d'assurer l'étanchéité du corps tubulaire en composite, à des pressions élevées, à tous les fluides, liquides ou gaz, d'une part et de permettre au piston de se déplacer à l'intérieur du corps tubulaire tout en assurant l'étanchéité entre lesdits piston et corps tubulaire par interposition de joints d'étanchéité montés selon les normes classiques, d'autre part.

Un autre avantage est que le corps tubulaire peut être conçu pour résister uniquement aux contraintes radiales, les tirants munis de butoirs encaissant les contraintes longitudinales, ce qui permet d'éviter de donner à la couche externe du corps tubulaire une épaisseur trop importante. De ce fait, il peut être rendu très léger et transparent aux rayons X ou gamma.

Un autre avantage est que le dépôt des pistons dégage complètement le corps de cellule, rendant la mise en place de la carotte extrêmement facile.

Un autre avantage encore est que la longueur de la cellule peut être adaptée aux impératifs de l'expérience moyennant la disponibilité de plusieurs corps tubulaires composites interchangeables et des couples de tirants adéquats.

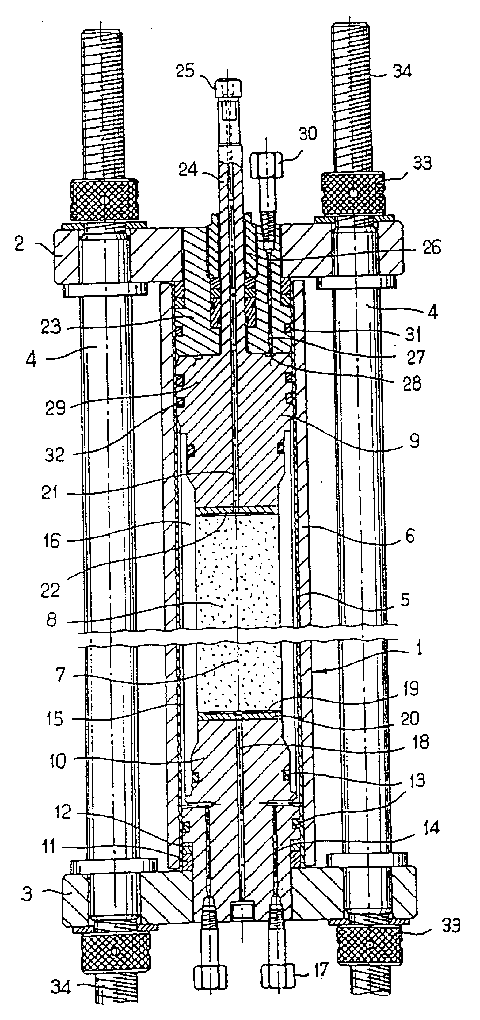

D'autres avantages et caractéristiques ressortiront mieux à la lecture de la description d'un mode de réalisation selon l'invention, ainsi que du dessin annexé sur lequel la figure unique est une vue en coupe longitudinale du dispositif selon l'invention.

Le dispositif comprend un corps tubulaire 1 disposé entre deux brides 2 et 3 qui sont reliées par 2 tirants 4.

Le corps tubulaire 1 est constitué par une chemise interne métallique 5, de faible épaisseur et d'un enrobage externe 6 qui est réalisé à partir de filaments de carbone imprégnés de résine du type époxy, et enroulés sur la chemise interne 5 suivant un angle d'environ 80°, par rapport à l'axe longitudinal 7 dudit corps tubulaire 1. L'enroulement change de direction à chaque extrémité, de manière à obtenir des filaments qui s'entrecroisent d'une couche à l'autre dans l'enrobage externe 6.

Dans le corps tubulaire 1 est disposé un corps poreux à tester 8, par exemple une carotte provenant d'un champs pétrolifère. De part et d'autre de la carotte 8 sont disposés deux pistons 9 et 10 dont l'un, 9, est mobile et l'autre, 10, fixe dans le corps tubulaire 1, le piston fixe 10 constituant en fait un bouchon d'entrée. Le bouchon d'entrée 10 est calé sur la bride 3 au moyen d'une rondelle 11 et comprend un joint 12 monté sur la rondelle 1 ainsi que des joints d'étanchéité toriques 13. Un ou plusieurs alésages 14 sont ménagés dans le bouchon d'entrée 10 et débouchent dans un espace annulaire 15 ménagé entre la chemise interne 5 et une membrane 16, par exemple en caoutchouc sous forme d'une gaine triple, qui enveloppe la carotte 8 et qui est fixée entre et sur le piston mobile 9 et le bouchon d'entrée 10. Des embouts 17 raccordent une source de fluide, non représentée, aux alésages 14, le fluide pouvant être par exemple de l'eau qui est utilisée pour plaquer la membrane 16 contre la carotte 7. Un alésage central 18 est également ménagé dans le bouchon d'entrée 10 pour permettre une injection d'un fluide ou des fluides de production du champ pétrolifère dans la carotte 8 par l'intermédiaire de rainures 19 ménagées sur la face 20 du bouchon d'entrée 10 ou sur une pièce intermédiaire montée entre la carotte 8 et le bouchon d'entrée 10.

Le piston mobile 9 comprend également un alésage central 21 pour l'injection de fluides de production par exemple dans la carotte 8, par l'intermédiaire de rainures 2. Un bouchon 23 est monté sous le piston mobile 9 et il est calé sur la bride 2 par un joint 12 et une rondelle 1, ledit bouchon 23 étant traversé par une queue 24 du piston mobile 9, et pourvue d'un embout 25. Une vis presse-étoupe 26 est traversée par la queue de piston 24 et prend appui sur un épaulement du bouchon 23, avec interposition de rondelle et joints d'étanchéité. Un alésage 27 est ménagé dans le bouchon 23 et relie, par un raccord 30, une source de fluide sous pression, non représentée, à une rainure annulaire 28 ménagée sur la face adjacente 29 du piston mobile 9, de manière à permettre la mise sous contrainte longitudinale de la carotte 8. Des joints toriques 31 et 32 assurent l'étanchéité respectivement du bouchon 23 et du piston mobile 9 vis-à-vis de la chemise interne 5.

Enfin, des écrous moletés 33 sont montés sur des parties filetées 34 des tirants 4 et permettent d'ajuster le dispositif de mesure à la longueur de la cellule de mesure qui est essentiellement constituée par le corps tubulaire 1.

Dans une forme de réalisation préférée de l'invention, la chemise interne 5 est réalisée en aluminium, et présente une épaisseur de 1 mm, ce qui ne constitue pas un obstacle à la transmission du rayonnement gamma et ce, en raison du faible coefficient d'absorption qui est environ 4 fois moins que l'acier et de sa faible épaisseur. Les expériences réalisées avec le dispositif selon l'invention ont permis de constater que la pression d'épreuve était de l'ordre de 600 bars avec une pression de service de l'ordre de 400 bars, la pression d'éclatement calculée étant de 2200 bars, alors que la température d'utilisation était d'environ 110°C.

On peut également réaliser la chemise intérieure 5 en acier à condition de diminuer l'épaisseur pour la rendre transparente aux rayons gamma, par exemple. Une épaisseur adéquate de la chemise intérieure en acier 5 est choisie entre 1/10 et 5/10 de mm.

Un procédé de fabrication du corps tubulaire 1 consiste, dans une première étape, à enrouler les filaments de carbone qui sont liés par une résine époxy appropriée, autour d'un mandrin d'aluminium, jusqu'à l'obtention de l'épaisseur souhaitée pour la couche externe composite, désignée par la référence 6 sur la figure. L'enroulement des filaments de carbone est réalisé suivant un angle qui, de préférence, est inférieur à 90° et, par exemple, compris entre 60 et 80°. De plus, à chaque extrémité du mandrin, l'enroulement change de direction de façon que deux couches adjacentes réalisées autour du mandrin présentent des filaments de carbone qui s'entrecroisent.

Dans une deuxième étape, on fore le mandrin d'aluminium jusqu'à l'obtention d'une chemise intérieure ou "Liner" d'épaisseur d'environ 1 mm. Après quoi, on rectifie, si nécessaire, la paroi interne de la chemise intérieure afin de permettre la mise en place correcte des bouchons et du piston avec leurs joints d'étanchéité, comme cela a été décrit précédemment.

Dans un exemple de réalisation donnant d'excellents résultats, la couche composite 6 présente une épaisseur sensiblement égale à 7,5 cm, et le corps tubulaire une longueur de 123 cm, avec un diamètre interne de la chemise de 6,9 cm.

Le corps tubulaire 1 obtenu selon le procédé de l'invention présente le grand avantage d'éviter une chemise interne qui soit en composite qui, en général, n'est pas étanche aux fluides, liquides ou gaz, et qui présente à ces fluides une certaine perméabilité conduisant à des fuites par "perlage".

Le fonctionnement du dispositif selon l'invention est le même que celui des dispositifs antérieurs, à la différence que lorsqu'on souhaite mettre la carotte sous contrainte longitudinale, on introduit un fluide approprié dans l'alésage 27, qui, en exerçant une pression sur la face 29 du piston 9, déplace ce dernier dans le corps tubulaire d'une course adéquate en vue de créer la contrainte longitudinale souhaitée.

## Revendications

1. Dispositif pour la mesure des écoulements fluidiques à travers un échantillon poreux (8), le dispositif comprenant un corps tubulaire (1) en deux parties dont la partie externe (6) est perméable aux rayons gamma, un support fixe d'échantillon (10) disposé à une autre extrémité du corps (1), une gaine souple (16) disposée à l'intérieur du corps tubulaire (1), l'échantillon étant disposé dans la gaine (16), des moyens d'injection (21, 22) d'au moins un fluide à une extrémité du corps tubulaire et à travers ledit échantillon (8), caractérisé en ce que ledit corps tubulaire (1) est revêtu sur sa paroi latérale d'une chemise métallique (5) de faible épaisseur et perméable audit rayonnement formant la partie interne, le corps tubulaire étant maintenu entre des tirants de maintien (4), le dispositif comprenant de plus un piston (9) introduit en partie à l'autre extrémité dudit corps tubulaire, l'échantillon étant disposé entre le piston (9) et le support (10).

2. Dispositif selon la revendication 1, caractérisé en ce que la chemise interne (5) est réalisée en aluminium.

3. Dispositif selon la revendication 2, caractérisé en ce que l'épaisseur de la chemise interne (5) est d'environ 1 mm.

4. Dispositif selon la revendication 1, caractérisé en ce que la chemise interne (5) est en acier et d'épaisseur comprise entre un et cinq dixièmes de mm.

5. Dispositif selon la revendication 1, caractérisé en ce que le corps tubulaire (1) est constitué par un enroulement de filaments liés par une résine, et en ce que les filaments sont enroulés suivant un angle de 80°.

6. Dispositif selon la revendication 1, caractérisé en ce que le piston (9) est mobile dans le corps tubulaire (1).

7. Dispositif selon la revendication 1, caractérisé en ce qu'un bouchon (23) est disposé sur la face du piston (9) qui est opposée à celle en contact avec l'échantillon (8), le bouchon (23) étant muni d'un passage 27 pour un fluide de mise en compression longitudinale du piston (9) le passage (27) débouchant dans une rainure annulaire (28) ménagée dans la face du piston (9) en regard du bouchon (23).

8. Procédé de fabrication du corps tubulaire pour le dispositif, suivant la revendication 1, caractérisé en ce qu'il consiste à enrouler les filaments sur un mandrin métallique ou sur un tube sur une épaisseur appropriée, puis à aléser ledit mandrin ou ledit tube sur toute la longueur jusqu'à l'obtention d'une chemise interne d'épaisseur adéquate pour être perméable à un rayonnement.

9. Procédé selon la revendication 8, caractérisé en ce que les filaments sont enroulés suivant un angle d'environ 30° et en ce que l'enroulement est alterné d'une couche à l'autre.

10. Procédé selon la revendication 8, caractérisé en ce que le mandrin ou le tube est en aluminium, et en ce que l'alésage est effectué jusqu'à l'obtention d'une chemise interne dont l'épaisseur est d'environ un millimètre.

## Patentansprüche

1. Vorrichtung zur Messung von Fluidströmen durch eine poröse Probe (8) mit
- einem rohrförmigen Körper (1) in zwei Teilen, deren äußerer Teil (6) für Gammastrahlen durchlässig ist,
- einem feststehenden Probenträger (10), der an einem anderen Ende des Körpers (1) angeordnet ist,
- einer biegsamen Hülle (16), die im Inneren des rohrförmigen Körpers (1) angeordnet ist, wobei die Probe in der Hülle (16) angeordnet ist,
- Einrichtungen (21, 22) zum Einspritzen wenigstens eines Fluids an einem Ende des rohrförmigen Körpers und durch die Probe (8),
dadurch gekennzeichnet, daß der rohrförmige Körper (1) über seiner Seitenwand mit einem Metallmantel (5) von geringer Dicke verkleidet ist, der für Strahlung durchlässig ist und den inneren Teil bildet, wobei der rohrförmige Körper zwischen Haltestangen (4) gehalten ist, wobei die Vorrichtung außerdem einen am anderen Ende des rohrförmigen Körpers teilweise eingeführten Kolben (9) aufweist, wobei die Probe zwischen dem Kolben (9) und dem Träger (10) angeordnet ist.

2. Vorrichtung nach Anspruch 1, dadurch gekennzeichnet, daß der Innenmantel (5) aus Aluminium hergestellt ist.

3. Vorrichtung nach Anspruch 2, dadurch gekennzeichnet, daß die Dicke des Innenmantels (5) umgefähr 1 mm beträgt.

4. Vorrichtung nach Anspruch 1, dadurch gekennzeichnet, daß der Innenmantel (5) aus Stahl ist und eine Dicke zwischen einem und fünf Zehntel mm hat.

5. Vorrichtung nach Anspruch 1, dadurch gekennzeichnet, daß der rohrförmige Körper (1) durch eine Wicklung von durch ein Harz verbundenen Filamenten gebildet ist und daß die Filamente unter einem Winkel von 80° gewickelt sind.

6. Vorrichtung nach Anspruch 1, dadurch gekennzeichnet, daß der Kolben (9) in dem rohrförmigen Körper (1) beweglich ist.

7. Vorrichtung nach Anspruch 1, dadurch gekennzeichnet, daß ein Verschlußstück (23) auf der Fläche des Kolbens (9) angeordnet ist, die von der in Kontakt mit der Probe (8) stehenden abgewandt ist, wobei das Verschlußstück (23) mit einem Durchgang (27) für ein Fluid zur Längskomprimierung des Kolbens (9) versehen ist, wobei der Durchgang (27) in einer Ringnut (28) mündet, die in der dem Verschlußstück (23) zugewandten Fläche des Kolbens (9) ausgespart ist.

8. Verfahren zur Herstellung des rohrförmigen Körpers für die Vorrichtung gemäß Anspruch 1, dadurch gekennzeichnet, daß die Filamente über eine geeignete Dicke auf einen Metalldorn oder auf ein Rohr gewickelt werden und dann der Dorn oder das Rohr über die gesamte Länge auf eine Dicke ausgebohrt werden, bei der der Innenmantel strahlungsdurchlässig ist.

9. Verfahren nach Anspruch 8, dadurch gekennzeichnet, daß die Filamente unter einem Winkel von ungefähr 30° gewickelt werden und daß die Wicklung von einer Schicht zur nächsten alterniert.

10. Verfahren nach Anspruch 8, dadurch gekennzeichnet, daß der Dorn oder das Rohr aus Aluminium sind und daß der Innenmantel bis auf eine Dicke von ungefähr 1 mm ausgebohrt wird.

## Claims

1. Device for measuring the flow of fluids through a porous sample (8), the device comprising a tubular member (1) in two sections of which the outer section (6) is permeable to gamma rays, a fixed sample support (10) disposed at a second end of the member (1), a flexible sheathing (16) disposed inside the tubular member (1), the sample being disposed in the sheathing (16), means (21, 22) for injecting at least one fluid at one end of the tubular member and through the said sample (8), characterised in that the said tubular member (1) is covered on its lateral wall with a thin metal lining (5) which is permeable to the said radiation, forming the inner section, the tubular member being held between support tie rods (4), the device further comprising a piston (9) partially inserted at the other end of the said tubular member, the sample being disposed between the piston (9) and the support (10).

2. Device according to Claim 1,
characterised in that the inner lining (5) is made of aluminium.

3. Device according to Claim 2,
characterised in that the thickness of the inner lining (5) is approximately 1 mm.

4. Device according to Claim 1,
characterised in that the inner lining (5) is made of steel and is between one and five tenths of a mm thick.

5. Device according to Claim 1,
characterised in that the tubular member (1) consists of a roll of filaments bonded by a resin; and in that the filaments are wound at an angle of 80°.

6. Device according to Claim 1,
characterised in that the piston (9) can move in the tubular member (1).

7. Device according to Claim 1,
characterised in that a plug (23) is disposed on the face of the piston (9) which is opposite the face which is in contact with the sample (8), the plug (23) being provided with a passage (27) for a fluid which longitudinally compresses the piston (9), the passage (27) opening out into an annular groove (28) provided in the face of the piston (9) opposite the plug (23).

8. Process for manufacturing the tubular member for the device according to Claim 1,
characterised in that it consists in winding the filaments onto a metal mandrel or onto a tube over a suitable thickness, then in boring the said mandrel or the said tube over the entire length until an inner lining of a thickness suitable for being permeable to radiation is obtained.

9. Process according to Claim 8,
characterised in that the filaments are wound at. an angle of approximately 30°; and in that the winding is alternated from one layer to another.

10. Process according to Claim 8,
characterised in that the mandrel or tube is made of aluminium; and.in that the hole is bored until an inner lining is obtained which is approximately 1 mm thick.
